# EUROPEAN PATENT SPECIFICATION

(11) **EP 2 473 848 B1**
(45) Date of publication and mention of the grant of the patent: **17.01.2018**
(21) Application number: 10754972.7
(22) Date of filing: 06.09.2010
(51) Int. Cl.: G01N 33/487

(54) **METHOD AND DEVICE TO IDENTIFY A TYPE OF TEST STRIP**
VERFAHREN UND VORRICHTUNG ZUR IDENTIFIZIERUNG EINES SENSORSTRIPS
PROCÉDÉ ET APPAREIL POUR L'IDENTIFICATION D'UNE BANDELETTE DE TEST

(30) Priority: 04.09.2009 US 240133 P
(43) Date of publication of application: 11.07.2012
(73) Proprietor: Lifescan Scotland Limited, Inverness IV2 3ED (GB)
(72) Inventor: WELSH, Raymond, Dunfermline KY12 9AU (GB); GUTHRIE, Brian, Inverness IV2 6RG (GB); DE ANGELI, Marco, I-24030 Barzana (IT); MACRAE, Allan, Inverness IV2 3RG (GB)
(74) Representative: Brunner, John Michael Owen
(86) International application number: PCT/GB2010/001684
(87) International publication number: WO 2011/027130

(56) References cited:
- EP-A1- 1 431 758
- EP-A2- 2 020 596
- WO-A1-2009/031761
- US-A1- 2003 042 150
- US-A1- 2003 150 724
- US-A1- 2006 099 703
- US-A1- 2008 159 911
- US-A1- 2009 095 623
- US-B1- 6 773 671

## Description

### CROSS-REFERENCE

This application claims the benefits of priority under 35 USC§ 119 and/or §120 from prior filed U.S. Provisional Application Serial No. 61/240,133 filed on September 4, 2009.

### BACKGROUND

The determination (e.g., detection, evaluation or calculation of the value or concentration measurement) of an analyte in a fluid sample is of particular interest in the medical field. For example, it may be desirable to determine glucose, cholesterol, acetaminophen and/or HbAlc concentrations in a sample of a bodily fluid such as urine, blood or interstitial fluid. Such determinations may be achieved using analyte test strips, based on, for example, photometric or electrochemical techniques, along with an associated test meter.

Typical electrochemical-based analyte test strips employ a plurality of electrodes (e.g., a working electrode and a reference electrode) and an enzymatic reagent to facilitate an electrochemical reaction with an analyte of interest and, thereby, determine the concentration of the analyte. For example, an electrochemical-based analyte test strip for the determination of glucose concentration in a blood sample can employ an enzymatic reagent that includes the enzyme glucose oxidase and the mediator ferricyanide. Such conventional analyte test strips are described in, for example, U.S. Patent Nos. 5,708,247; 5,951,836; 6,241,862; and 6,284,125. Measuring instruments that use an electrochemical cell as are typically provided by a disposable test strip or the like are well known and popular with consumers. These instruments are used for the detection of various analyte levels in physiological fluid samples. For example, the concentration of an analyte in a variety of different physiological samples, such as urine, tears, saliva, and the like may be determined with these instruments. One popular application is for determining the concentration of an analyte in interstitial fluid, blood or blood fractions, and more particularly in whole blood.

### SUMMARY OF THE DISCLOSURE

The invention is directed to a method of differentiating between two types of analyte test strips using a transistor switch and a microcontroller, the method comprising the steps defined in claim 1. The invention is also directed to a glucose meter according to claim 8.

### BRIEF DESCRIPTION OF THE DRAWINGS

The accompanying drawings, which are incorporated herein and constitute part of this specification, illustrate presently preferred embodiments of the invention, and, together with the general description given above and the detailed description given below, serve to explain features of the invention.
Figure 1 illustrates an analyte measurement and management device that can identify a particular type of test strip.
Figure 2 illustrates a top portion of an exemplary circuit board of the analyte measurement and management device for the device of Figure 1.
Figure 3 illustrates a bottom portion of the circuit board of the analyte measurement and management device for the device of Figure 1.
Figure 4 illustrates a simplified exploded perspective view of a first type of test strip.
Figure 5 illustrates an arrangement of layers of the first type of test strip of Figure 4.
Figure 6 illustrates an arrangement of layers of a second type of test strip.
Figure 7 illustrates a plurality of connector points that correspond to the contact pads of the first type of test strip of Figure 4.
Figure 8 illustrates a plurality of connector points that correspond to the contact pads of the second type of test strip of Figure 6.
Figure 9 illustrates alternative test strip embodiments having various contact pad patterns.
Figure 10 illustrates electronic circuitry for identifying a first type of test strip having a particular pattern of contact pads.
Figure 11 illustrates the electronic circuitry of Figure 10 for identifying a second type of test strip having a particular pattern of contact pads.
Figure 12A illustrates a strip discrimination line for controlling a switch that probes for the identification of the type of test strip
Figure 12B illustrations a strip detection line that monitors the high and low logic states with time as a result of the toggling of the switch with a first type of test strip.
Figure 13 illustrations a strip detection line that monitors the high and low logic states with time as a result of the toggling of the switch with a second type of test strip.
Figure 14 illustrations a strip detection line that monitors the high and low logic states with time as a result of the toggling of the switch with a test strip that is inserted, removed, and re-inserted.
Figure 15 is a flow chart illustrating top level logic to recognize a test strip insertion and then identify the type of test strip.
Figure 16 is a flow chart illustrating the process in more detail to determine the type of test strip.
Figure 17 is a flow chart illustrating the process in more detail to attempt the determination of the type of test strip.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict selected exemplary embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. In addition, as used herein, the terms "patient," "host," "user," and "subject" refer to any human or animal subject and are not intended to limit the systems or methods to human use, although use of the subject invention in a human patient represents a preferred embodiment.

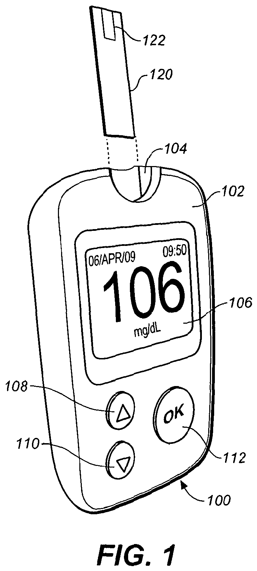

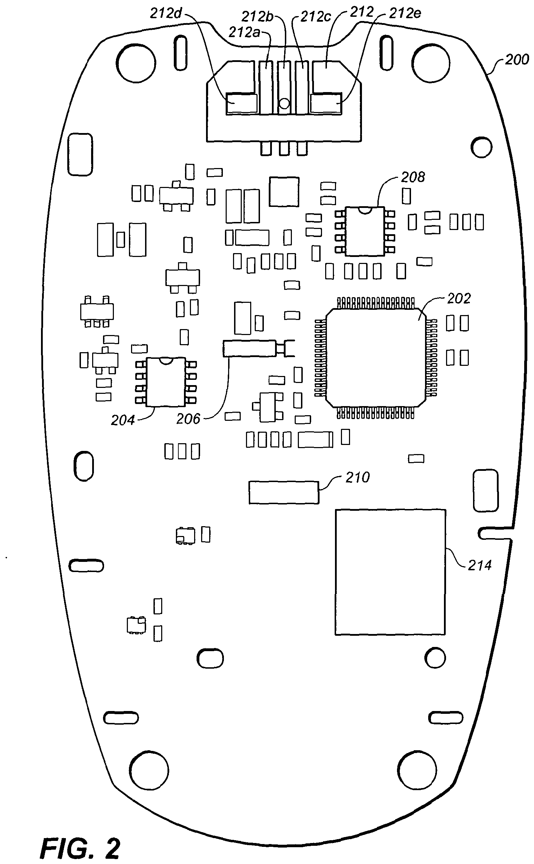

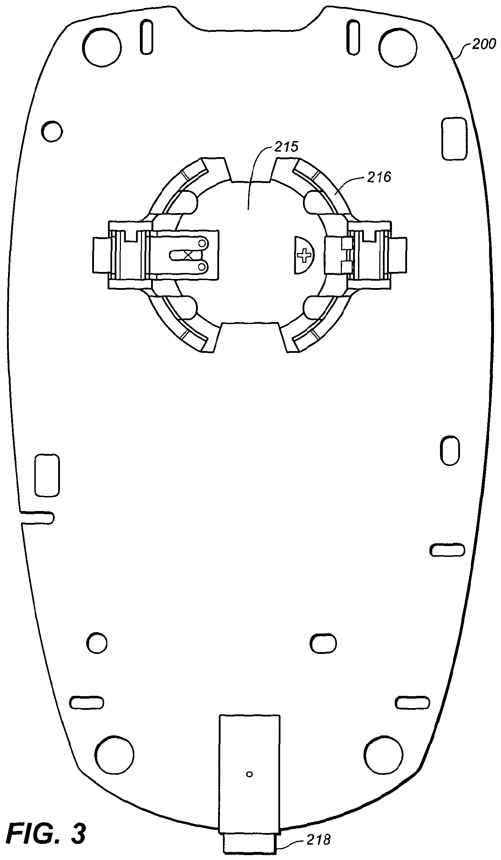

Figure 1 illustrates a diabetes management system that includes a blood glucose measurement unit 100 with a blood glucose test strip 120 having an inlet port 122 to receive a blood sample. The blood glucose measurement device 100 includes a housing 102 with a test strip port 104 configured to receive the test strip and a display 106 along with user interface buttons 108, 110, and 112. Disposed inside housing 102 includes, as shown in Fig. 2, a circuit board 200 with a microcontroller 202 coupled to a memory 204, clock 206, operational amplifier 208, and display connector 210. The op-amp 208 and microcontroller 202 are operatively connected to a strip port connector 212 with contacts 212a, 212b, 212c, 212d, and 212e (first, second, third, fourth, and fifth, respectively) for mechanical contact with corresponding contact pads on the test strip 120. To facilitate communication with other data management devices, a wireless transceiver module 214 is provided to allow for bi-directional communication of data stored in the memory 204 of the unit 100. On the other side of circuit board 200, shown here in Fig. 3, a power source in the form of a battery 215 is provided. A data port 218 may also be provided. It should be noted that the unit 100 is preferably sized and configured to be handheld and the transceiver 214 may be for use with either or both of a short-range wireless network (e.g., BlueTooth, Zigbee or Wi-Fi and the like) or a longer range wireless network (e.g., GSM, CDMA, 3G and the like).

Referring back to Figure 2, microcontroller 202 may be electrically connected to strip port connector 212, operational amplifier circuit 208, first wireless module 214, display 106, non-volatile memory 204, clock 206, battery connector 216, data port 218, and user interface buttons (108, 110, and 112). Specifically, user interface buttons (108, 110, and 112) include a first user interface button 108, a second user interface button 110, and a third user interface button 112. Data entered can include values representative of analyte concentration, or in the context of the analyte concentration values coupled with information, which are related to the everyday lifestyle of an individual. Information, which is related to the everyday lifestyle, can include food intake, medication use, occurrence of health check-ups, and general health condition and exercise levels of an individual coupled to or "tagged" with the analyte concentration value of the user at specific time of the day or week.

Operational amplifier circuit 208 may be two or more operational amplifiers configured to provide a portion of the potentiostat function and the current measurement function. The potentiostat function can refer to the application of a test voltage between at least two electrodes of a test strip. The current function can refer to the measurement of a test current resulting from the applied test voltage to the test strip 120. The current measurement may be performed with a current-to-voltage converter. Microcontroller 202 may be in the form of a mixed signal microprocessor (MSP) such as, for example, the Texas Instrument MSP430F2419. The TI-MSP430F2419 may be configured to also perform a portion of the potentiostat function and the current measurement function. In addition, the MSP430F2419 can also include volatile and non-volatile memory. In another embodiment, many of the electronic components may be integrated with the microcontroller in the form of an application specific integrated circuit (ASIC).

Referring back to Figure 2, strip port connector 212 may be configured to form an electrical connection to the test strip. Display connector 210 may be configured to attach to display 106. Display 106 may be in the form of a liquid crystal display for reporting measured glucose levels, and for facilitating entry of lifestyle related information and for manipulation of graphical data, pictorial results and motion video, as illustrated in Figure 2. Display 106 may also include a backlight.

Referring back to Figure 3, data port 218 can accept a suitable connector attached to a connecting lead, thereby allowing meter unit 100 to be linked to an external device such as a personal computer. Data port 218 may be any port that allows for transmission of data such as, for example, a serial, USB, or a parallel port. Clock 206 may be configured for measuring time and be in the form of an oscillating crystal. Battery connector 216 may be configured to be electrically connected to battery 215.

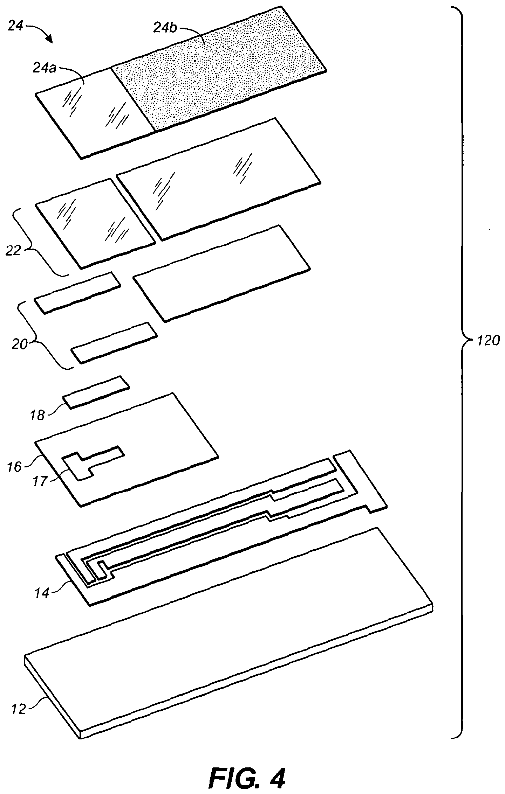

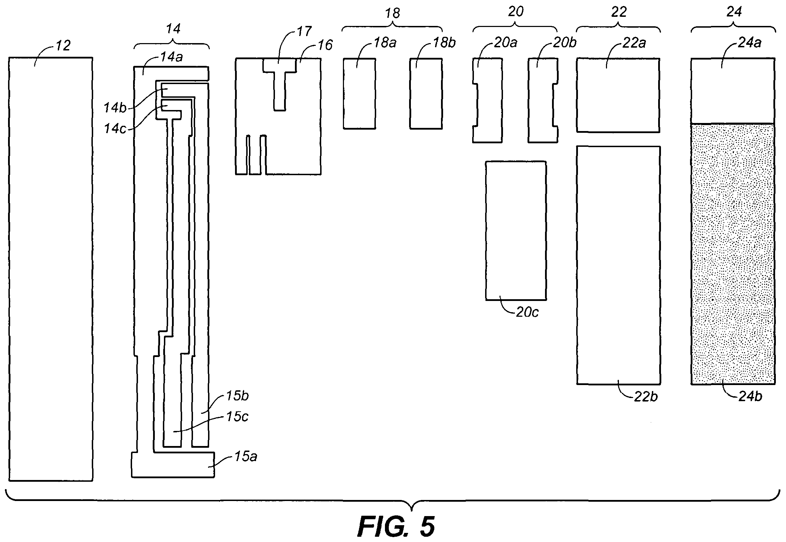

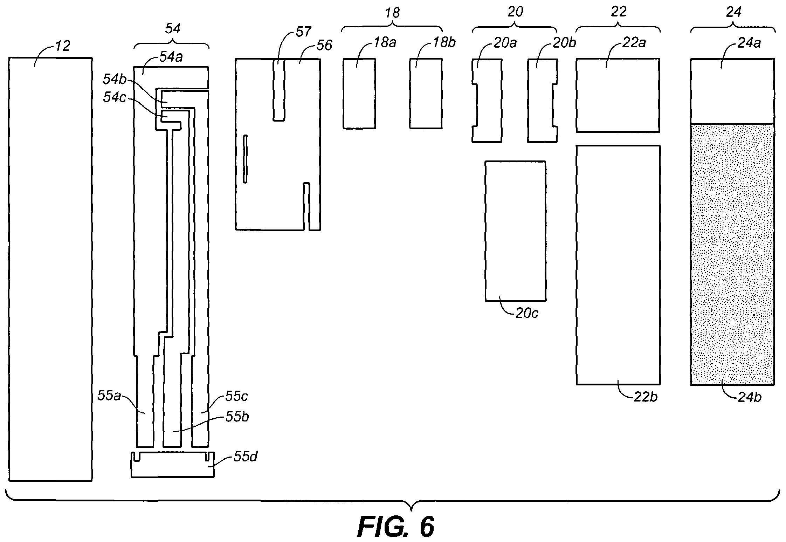

For use with the meter 100 is an exemplary test strip 120, as illustrated in Figure 4, that includes an electrically-insulating substrate 12, a patterned conductive layer 14 disposed over the electrically-insulating substrate 12, a patterned insulating layer 16 disposed over the patterned conductive layer 14, an enzymatic reagent layer 18 disposed at least over at least a portion of the patterned conductive layer 14, a patterned adhesive layer 20 disposed over at least a portion of the patterned insulating layer 16, a hydrophilic layer 22 disposed over the patterned adhesive layer 20, and a top layer 24 (with a first portion 24a and an opaque second portion 24b) disposed over the hydrophilic layer 22, which has two portions 22a and 22b. Patterned adhesive layer 20 can include three pads (20a, 20b, and 20c), as illustrated in Figures 5 and 6.

Figure 5 illustrates an arrangement of layers of a first type of test strip 120 of Figure 4. A type of test strip can refer to a particular brand of test strips configured to perform a glucose test with blood glucose monitor 100. In an embodiment, blood glucose monitor 100 may be configured to perform a glucose test with only with the first type of test strip (120). Patterned conductive layer 14 can include a reference electrode 14a, a first working electrode 14b, a second working electrode 14c, a reference contact pad 15a, a first contact pad 15b, and a second contact pad 15c. In an embodiment, a contact pad may also be referred to as a conductive track. As illustrated in Figures 4 and 5, reference electrode 14a, a first working electrode 14b, and a second working electrode 14c are each electrically connected to a reference contact pad 15a, a first contact pad 15b, and a second contact pad 15c, respectively. Note that the arrangement and geometry of the contact pads may be indicative of a particular brand or type of test strip. In an embodiment, contact pads (15a, 15b, and 15c) may be configured to electrically interface with corresponding contacts of the strip port connector.

Referring back to Figures 4 and 5, the disposition and alignment of patterning of electrically-insulating substrate 12, patterned conductor layer 14 (including reference electrode 14a, first working electrode 14b and second working electrode 14c), patterned insulation layer 16 (with electrode exposure window 17 extending therethrough) and enzymatic reagent layer 18, and patterned adhesive layer 20, hydrophilic layer 22, and top layer 24 of electrochemical-based analytical test strip 120 are such that a sample receiving-chamber is formed within electrochemical-based analytical test strip 120.

Electrochemical-based analytical test strip 120 may be manufactured, for example, by the sequential aligned formation of patterned conductor layer 14, patterned insulation layer 16 (with electrode exposure window 17 extending therethrough), enzymatic reagent layer 18, patterned adhesive layer 20, hydrophilic layer 22 and top film 24 onto electrically-insulating substrate 12. Any suitable techniques may be used to accomplish such sequential aligned formation, including, for example, screen printing, photolithography, photogravure, and chemical vapor deposition and tape lamination techniques.

During use of electrochemical-based analytical test strip 120 to determine an analyte concentration in a fluid sample (e.g., blood glucose concentration in a whole blood sample), electrodes 14a, 14b and 14c of patterned conductor layer 14 are employed to monitor an electrochemical reaction induced current of interest. The magnitude of such a current may then be correlated with the amount of analyte present in the fluid sample under investigation. During such use, a bodily fluid sample is introduced into sample-receiving chamber 26 of electrochemical-based analytical test strip 10.

Figure 6 illustrates an arrangement of layers of a second type of test strip 124. Test strip 124 may be similar to test strip 120 where test strip 124 has a different patterned conductive layer 54 and a different patterned insulation layer 56 having an electrode exposure window 57. In an embodiment, blood glucose monitor 100 may be configured to not perform a glucose test with the second type of test strip 124. In another embodiment, blood glucose monitor 100 may be configured to perform a glucose test with either the first or second type of test strip.

Referring back to Figure 6, patterned conductive layer 54 may include a reference electrode 54a, a first working electrode 54b, a second working electrode 54c, a reference contact pad 55a, a first contact pad 55b, and a second contact pad 55c. As illustrated in Figures 6, reference electrode 54a, a first working electrode 54b, and a second working electrode 54c are each electrically connected to a reference contact pad 55a, a first contact pad 55b, and a second contact pad 55c, respectively. In an embodiment, contact pads (15a, 15b, and 15c) may be configured to electrically interface with corresponding contacts of the strip port connector.

Electrically-insulating substrate 12, which is common to both the first type of strip 120 and the second type of strip 124, may be a nylon substrate, polycarbonate substrate, a polyimide substrate, a polyvinyl chloride substrate, a polyethylene substrate, a polypropylene substrate, a glycolated polyester (PETG) substrate, or a polyester substrate. The electrically-insulating substrate may have any suitable dimensions including, for example, a width dimension of about 5 mm, a length dimension of about 27 mm and a thickness dimension of about 0.5 mm.

Electrically-insulating substrate 12 provide structure to the strip for ease of handling and also serves as a base for the application (e.g., printing) of subsequent layers (e.g., a carbon-based patterned conductive layer). It should be noted that patterned conductor layers employed in analytical test strips may take any suitable shape and be formed of any suitable materials including, for example, metal materials and conductive carbon materials.

Patterned conductive layer 14 includes a counter electrode 14a (also referred to as a reference electrode), a first working electrode 14b, and a second working electrode 14c (see Figures 4 and 5). Although test strip 120 is depicted as including three electrodes, embodiments of electrochemical-based analytical test strips may include any suitable number of electrodes.

Counter electrode 14a, first working electrode 14b and second working electrode 14c may be formed of any suitable material including, for example, gold, palladium, platinum, indium, titanium-palladium alloys and electrically conducting carbon-based materials. Details regarding the use of electrodes and enzymatic reagent layers for the determination of the concentrations of analytes in a fluid sample are in U.S. Patent No. 6,733,655.

Patterned insulation layer 16 may be formed, for example, from a screen printable insulating ink. Such a screen printable insulating ink is commercially available from Ercon of Wareham, Massachusetts U.S.A. under the name "Insulayer."

Patterned adhesive layer 20, which may be common for both strip 120 and strip 124, may be formed, for example, from a screen-printable pressure sensitive adhesive commercially available from Apollo Adhesives, Tamworth, Staffordshire, UK. In the embodiment of Figures 4 and 5, patterned adhesive layer 20 defines outer walls of the sample-receiving chamber 26.

Hydrophilic layer 22, which may be common for both strip 120 and strip 124, may be, for example, a clear film with hydrophilic properties that promote wetting and filling of electrochemical-based analytical test strip 120 by a fluid sample (e.g., a whole blood sample). Such clear films are commercially available from, for example, 3M of Minneapolis, Minnesota U.S.A.

Enzymatic reagent layer 18, which may be common for both strip 120 and strip 124, may include any suitable enzymatic reagents, with the selection of enzymatic reagents being dependent on the analyte to be determined. In an embodiment, two overlapping enzymatic reagent layers 18 may be printed over the conductive layer, which is illustrated in Figure 5, as first reagent layer 18a and second reagent layer 18b. For example, if glucose is to be determined in a blood sample, enzymatic reagent layer 18 may include an enzyme and a mediator, along with other components necessary for functional operation. Enzymatic reagent layer 18 may include, for example, glucose oxidase, tri-sodium citrate, citric acid, polyvinyl alcohol, hydroxyl ethyl cellulose, potassium ferricyanide, antifoam, cabosil, PVPVA, and water.

Exemplary enzymes suitable for use in the reagent layer include glucose oxidase, glucose dehydrogenase (with pyrroloquinoline quinone co-factor, "PQQ"), and glucose dehydrogenase (with flavin adenine dinucleotide co-factor, "FAD"). An exemplary mediator suitable for use in the reagent layer includes ferricyanide, which in this case is in the oxidized form. The reagent layer may be configured to physically transform glucose into an enzymatic by-product and in the process generate an amount of reduced mediator (e.g., ferrocyanide) that is proportional to the glucose concentration value. Further details regarding enzymatic reagent layers, and electrochemical-based analytical test strips in general, are in U.S. Patent No. 6,241,862.

Top layer 24, which may be common for both strip 120 and strip 124, includes a first portion 24a (e.g. a transparent or translucent first portion) and an opaque second portion 24b. First portion 24a and the opaque second portion 24b of the top layer are configured and aligned with the remainder of the analytical test strip such that a user can view the working portion of the sample-receiving chamber through the first portion of the top layer and is precluded from viewing the non-working portion of the sample-receiving chamber by the opaque second portion of the top layer. This configuration prevents a user from erroneously determining that a sample fill error has occurred when the working portion of the sample-receiving chamber has been filled but the non-working portion has not been filled.

Top layer 24 may be, for example, a clear film, with opaque second portion 24b being created, for example, by overprinting of the clear film with an opaque ink and first portion 24a being simply clear film without overprinting. A suitable clear film is commercially available from Tape Specialties UK.

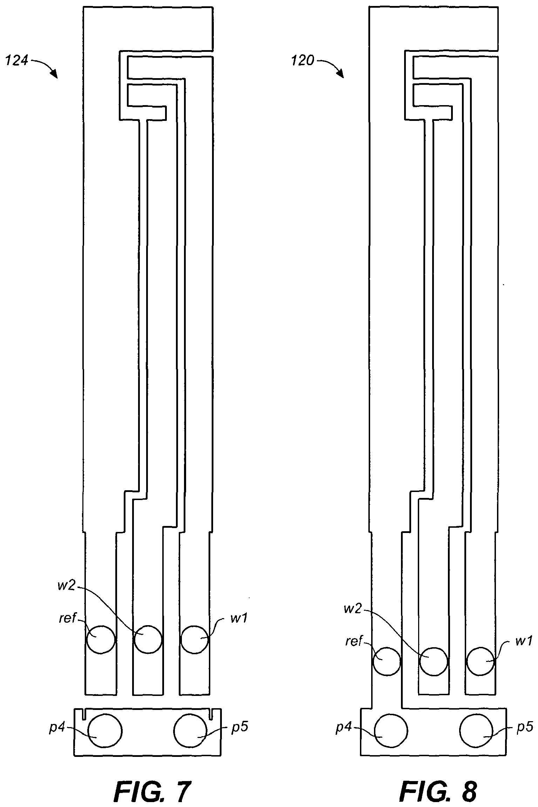

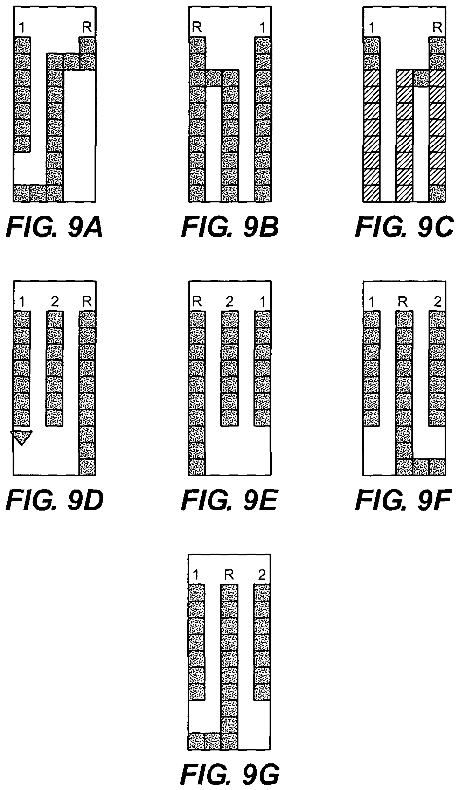

Figures 7 and 8 illustrate a plurality of strip port connector points, which are designated areas on the contact pads of the first and second type of test strips. Strip connector points may be referred to as **p4, ref, w2, w1,** and **p5.** Contacts 212d, 212a, 212b, 212c, and 212e of strip port connector 212 can make electrical contact with the contact pads at strip connector points **p4, ref, w2, w1,** and **p5,** respectively, when a test strip is inserted into the blood glucose meter. Note that the embodiments described herein should not be limited to those described in Figures 7 and 8 and could apply to a wide variety of contact pad patterns as illustrated in Figure 9 and referenced as patterns A-G.

Applicants recognized that there is a need for a blood glucose meter that can differentiate between different types of test strips. A blood glucose meter may be configured to perform a glucose test with a particular type of test strip, and if a different type of test strip is inserted, then the meter can output an error message. A method for determining a type of test strip should be robust so that an inadvertent withdrawal of the test strip or introduction of noise would not cause a misidentification.

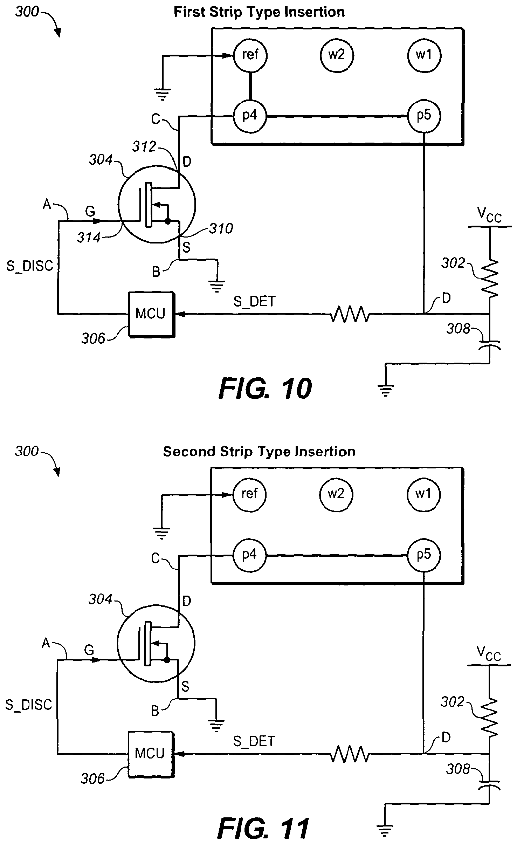

In an embodiment, electronic circuitry may be employed to differentiate between different types of test strips. Figure 10 illustrates electronic circuitry 300 for identifying a first type of test strip 120, which may be integrated onto circuit board 200. Electronic circuitry 300 can include a pull-up resistor 302, a capacitor 308, a switch 304, and a microcontroller 306. Pull-up resistor 302 may be connected to a supply voltage Vcc and limits the amount of current flowing through the electronic circuit when a strip is inserted into the blood glucose meter 100. Capacitor 308 is connected to the electronic circuitry to filter a measured voltage.

Switch 304 may be in the form of metal oxide semiconductor field effect transistor (e.g., MOSFET or FET). Microcontroller 306 may control the switch 304 for applying a voltage waveform and to measure the resulting signal. More specifically, microcontroller 306 may be configured to open and close the transistor switch 304 with a first interrupt and also to measure transitions in a logic state at a second interrupt so that the glucose meter 100 may identify a first or second type of test strip 120 or 124, respectively. A transistor switch may have a source input 310 connected to a ground, a drain input 312 connected to connector point **p4,** and a gate input 314 connected to the first interrupt of a microcontroller along a strip discriminate line (**S_DISC**).

Electronic circuitry 300 may be electrically connected to the contacts (212d, 212a, 212b, 212c, and 212e) of the strip port connector 212, which in turn, respectively connect to the connector points (**p4, ref, w2, w1,** and **p5**). A strip detect line (**S_DET**) may be a second interrupt to connector point **p5** and from microcontroller 306. A strip discriminate line (**S_DISC**) may be a first interrupt to switch 304. Switch 304 may be connected to reference connector point **p4,** microcontroller 306, and ground. The reference connector point **ref** may be connected to ground.

Referring back to Figure 10, switch 304 is initially closed while the meter is in a sleep mode and no strip has yet been inserted. At this point, the gate point A in the diagram is driven high. Also, points B and C are shorted and therefore connected to ground. Because connector points **p4** and **p5** are discontinuous, junction point D will remain high, which is limited by pull-up resistor 302 and the supply voltage Vcc.

When a first type of test strip 120 is inserted into the strip port connector 212 causing connector points **ref, p4,** and **p5** to short together, as illustrated in Figure 10. Thus, the strip insertion of strip 120 causes point D to be pulled down from supply voltage Vcc level to ground because connector points **p4** and **p5** are shorted together. The change of state on the **S_DET** pin on microcontroller 306 alerts the meter to turn on. Microcontroller 306 then toggles the gate to the FET switch 304 at point A to high and low for a number of pulses, which preferably are about 4 pulses. The toggling of switch 304 has the effect opening and closing the continuity between points B and C.

For a situation in which a first type of test strip 120 has been inserted, point D will always be held at a logic low (i.e., GND) while switch is opened and closed four times because of the connection through **p4** to **ref,** where **ref** is connected to GND. After the application of the pulses, microcontroller 306 will look to determine if there is a continual low level logic to ensure that the test strip has not been removed during the strip identification process. Where microcontroller 306 detects continual low level logic during the pulses and for a period of time thereafter, the software recognizes the presence of the first type of test strip 120 and initiates a blood glucose test by prompting a user to apply blood.

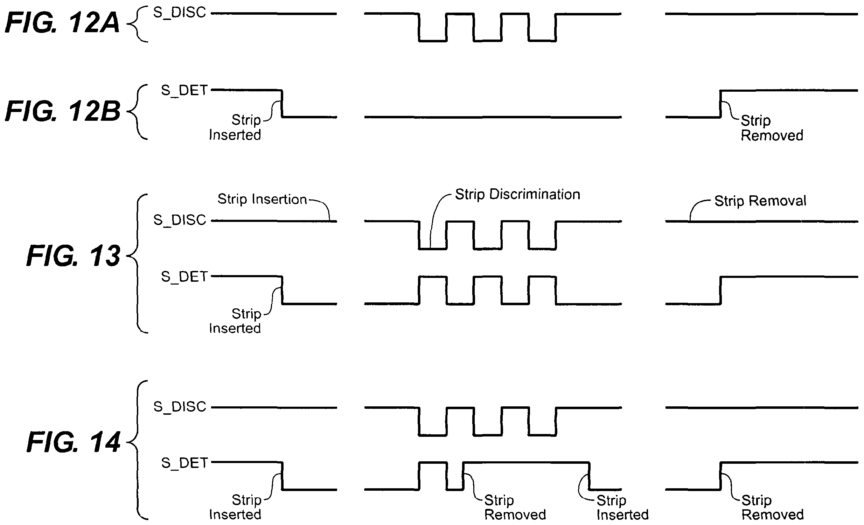

Figure 12A illustrates a strip discrimination line for controlling switch 304 that probes for the identification of the type of test strip. Initially, when no strip 120 is connected to the meter 100, the strip discrimination line logic remains high. At some point after a strip has been inserted and recognized, where **p4** and **p5** are shorted, the logic toggles between high and low. In the embodiment depicted in Figure 12A, there are four highs and three lows, however, other numbers of highs and lows may be used for identification of a type of test strip in a robust manner. In another embodiment, four highs and four lows may be used where each high and low pulse has a duration of about thirty milliseconds. In a preferred embodiment, the strip discrimination pulses generated are sent at a duration greater than about 2 seconds after the meter detects a strip insertion and completes its self checks.

The frequency of pulses has to be sufficiently high so that it cannot be replicated by a manual removal and insertion process by a user. The user may insert and remove a strip about 10 times per second (i.e., 100 milliseconds per cycle). Thus, in an embodiment, a pulse length may be less than 50 milliseconds so that the pulse length will be shorter than the manual process by about a factor of two. In an alternative embodiment, the pulse waveform may be asymmetric where the low pulse may be about 20 milliseconds and the high pulse may be about 5 milliseconds.

Figure 12B illustrates a strip detection line that monitors the high and low logic states with time as a result of the toggling of the switch with a first type of test strip 120. Once a first type of test strip 120 is inserted, the strip detection line transitions from high to low. Even when the strip discrimination line toggles between high and low, the strip detection line remains low. At some time after the toggling of the switch, microcontroller checks to make sure that the strip 120 has not been removed before initiating the glucose measurement process. In an embodiment, a debounce timer is disabled at the commencement of the discrimination test. The debounce timer may be any hardware or software utilized to ensure that a correct reading of the signal upon contact between the contact pads and the contacts of the strip port connector. A verification that the strip was not removed takes place by re-enabling the strip detect interrupt and monitoring a low logic state for about 200 milliseconds, which is about the duration of the debounce timer.

Figure 11 illustrates electronic circuitry 300 for identifying a second type of test strip 124. Switch 304 is initially closed while the meter is in a sleep mode and no strip has yet been inserted. At this point, the gate point A in the diagram is driven high. Also, points B and C are shorted and therefore connected to ground. Because connector points **p4** and **p5** are discontinuous, point D will remain high, which is limited by pull-up resistor 302 and the supply voltage VCC.

When a second type of test strip 124 is inserted into the strip port connector configured specifically for the first type of strip 120, this causes connector points **p4,** and **p5** to short together, as illustrated in Figure 11. Thus, the strip insertion of the second type of strip 124 causes point D to be pulled down from VCC level to GND because connector points **p4** and **p5** are shorted together. The change of state on the **S_DET** pin on microcontroller 306 alerts the meter to wake up because of the strip insertion. Once a strip insertion has been detected, microcontroller 306 then toggles the gate to the FET switch 304 at point A to high and low for a total of 4 pulses. The toggling of switch 304 has the effect of opening and closing the continuity between points B and C.

For the situation in which a second type of test strip 124 (instead of the first type of strip 120) has been inserted, the **S_DET** pin will read back the four pulses. The logic level will transition to high when switch 304 is open and transition to low when switch 304 is closed. Note, that in contrast to the insertion of the first type of test strip 120, point D will not always be held at a logic low (i.e. GND) because there is a discontinuous connection between **p4** to **ref.** Microcontroller will determine whether the **S_DET** pin measures the same number of pulses (e.g., four pulses) that were sent out by the **S_DISC** pin. If this condition is true, then microcontroller 306 may determine that a second type of test strip 124 has been inserted and output an error message, which may be by audio, visual or both audiovisual annunciation by the meter 100. However, if the **S_DET** pin measures a different number of pulses that were sent out by the **S_DISC** pin, then the process of sending four pulses is repeated two more times to detect the presence of the second type of test strip 124.

Figure 13 illustrations a strip detection line that monitors the high and low logic states with time as a result of the toggling of the switch with a second type of test strip 124. Once a second type of test strip 120 is inserted, the strip detection line transitions from high to low. However, when the strip discrimination line transitions from high to low, the strip detection line transition from low to high, and vice versa. At some time after the toggling of the switch, microcontroller 306 checks to make sure that the strip 120 has not been removed before initiating the glucose measurement process.

Under certain circumstances, removal and re-insertion of the test strip or environmental noise may cause the **S_DET** pin to measure a different number of pulses than that sent out by the **S_DISC** pin. Figure 14 illustrates a strip detection line that monitors the high and low logic states with time as a result of the toggling of the switch with a test strip that is inserted, removed, and re-inserted. The test strip is removed during the pulsing process causing the number of highs and lows to be observed on the strip detection line **S_DET** to be different than the strip discrimination line **S_DISC.** When this occurs, then the process of sending a suitable wave form such as, for example, a square wave is repeated two more times to see if the test strip type may be identified.

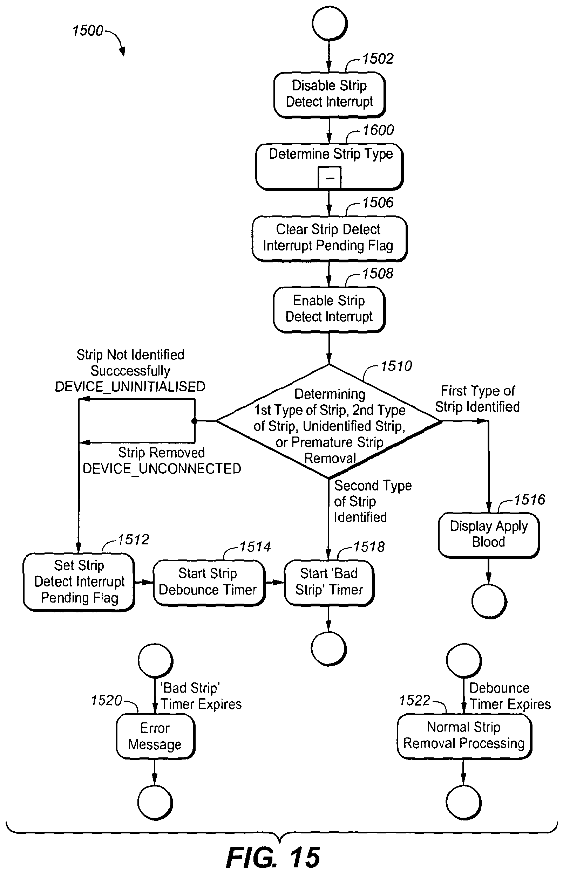

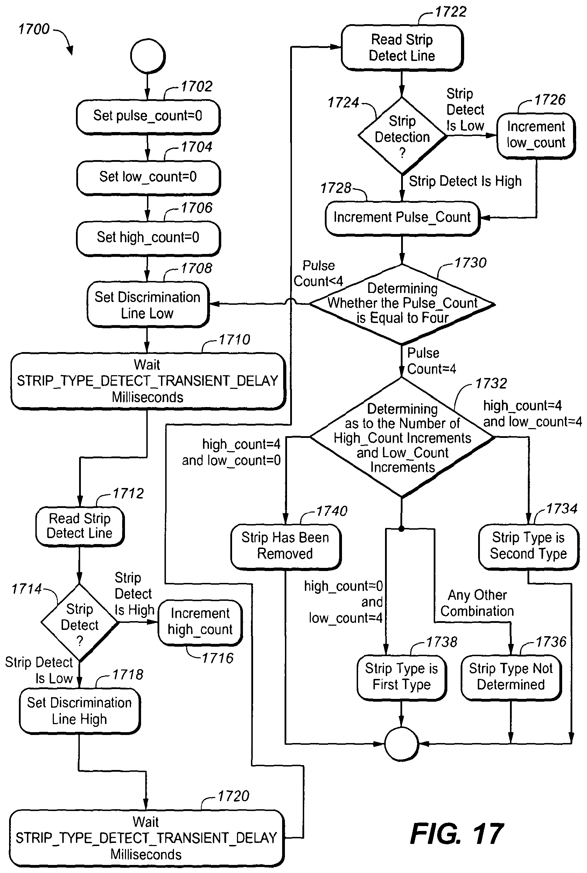

Figure 15 is a flow chart illustrating a top level logic 1500 to recognize a test strip insertion and then identify the type of test strip. Once a test strip insertion has been detected with a strip detect interrupt, the interrupt is disabled, as illustrated in a step 1502. The strip detect interrupt is disabled before the determination attempt (see sub-routine 1700 of Figure 17) and any pending interrupts are cleared after the determination attempt, but before the interrupt is re-enabled. This is because the action of pulsing the strip detect line will cause interrupts to be flagged as pending - if the pending interrupt was not cleared, this would cause the debounce logic to be fired unnecessarily. A pending interrupt may refer to an interrupt that is registered, but has not yet occurred. It should be noted that if the interrupts are re-enabled when there are pending flags, this will cause the interrupt service routine to be invoked resulting in the debounce logic being initiated.

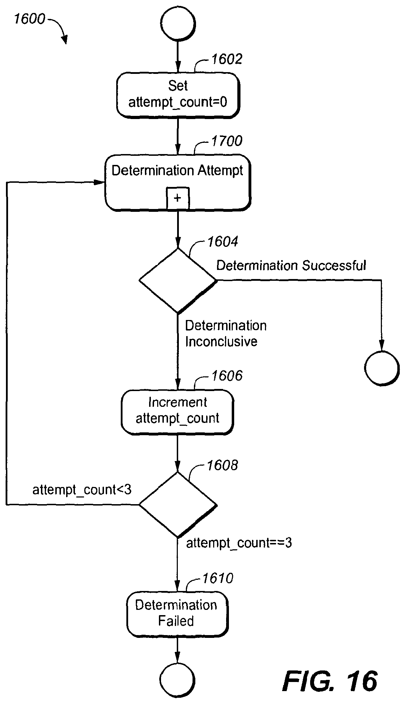

After step 1502, a sub-routine 1600 is performed, as illustrated in Figure 16. The strip detect interrupt is cleared of any pending flags, and then the strip detect interrupt is enabled, as illustrated in steps 1506 and 1508. For example, a microprocessor such as the MSP430 has an 'IFG' register, which latches any edge transitions detected on the strip detect input. Whether interrupts are enabled or disabled, the microprocessor can set this register to indicate "pending flags" on that input. Next, a determination is made as to whether a first type of strip was identified, a second type of strip was identified, the strip was not identified successfully, or the strip was removed prematurely, as illustrated in step 1510. Where a first type of strip was identified, then the blood glucose meter may display an apply blood prompt, as illustrated in a step 1516. Where a second type of strip was identified, then a 'bad strip' timer may be started, as illustrated in a step 1518. Where a strip was not identified successfully, or the strip was removed prematurely, then the strip detect interrupt pending flag may be set, the strip debounce timer, and the 'bad strip' timer may both be started, as illustrated in steps 1512, 1514, and 1518.

Note that in both the scenarios where the strip is not identified, but is determined to be present, and where the strip is determined not to be present, the strip removal debounce timer is started and a 'bad strip' timer is set to fire just after the debounce time expires. If this 'bad strip' timer event is received, an error message is displayed, as illustrated in a step 1520. If the debounce timer expires (which will happen before the 'bad strip' timer event if the strip has not been reinserted) and detects strip removal, then the normal strip removal event will be raised and handled before an error message is displayed, as illustrated in a step 1522. If, when the debounce timer expires, the strip is determined to be present, then the strip removal event will not be raised, and an error message will be displayed when the 'bad strip' timer event is received. This prevents the transient display of an error message when a strip is removed during the strip type detect operation.

Figure 16 is a flow chart illustrating a process in more detail to determine the type of test strip using sub-routine 1600. Initially, an **attempt_count** is set to zero, as illustrated in step 1602. Next, a **determination attempt** sub-routine is performed, as illustrated in step 1700. After sub-routine 1700, a determination is made as to whether the determination was successful or whether the determination was inconclusive, as illustrated in a step 1604. Where the determination was inconclusive, the **attempt_count** is incremented by one, as illustrated in a step 1606. The **attempt_count** is compared to a threshold value of three, in a step 1608. If the **attempt_count** is less than 3, then the steps 1700, 1604, and 1606 are repeated. If the **attempt_count** equals 3, then the determination fails, as illustrated in a step 1610.

Figure 17 is a flow chart illustrating the process in more detail to attempt the determination of the type of test strip. Initially, the **pulse_count,** the **low_count,** the **high_count** are all set to zero, and the discrimination line is set low, as illustrated in steps 1702, 1704, 1706, and 1708. Next, there is a wait time for a **strip_type**_**detect**_**transient**_**delay** (in milliseconds), as illustrated in a step 1710. In an embodiment, the **strip_type_detect_transient_delay** may be an amount of time that passes before doing a read step such as, for example, about thirty milliseconds. The delay time must be sufficiently long to allow the input pin to reach a logic high. After step 1710, the strip detect line is read by the microcontroller, as illustrated in a step 1712. A determination is made as to whether the strip detect line is high or low, as illustrated in a step 1714. If the strip detect is high, then the **high_count** is incremented by one, as illustrated in a step 1716. However, if the strip detect is low, then the discrimination line is set to high, as illustrated in a step 1718.

Next, there is a wait time for a **strip_type_detect_transient_delay** (in milliseconds), as illustrated in a step 1720. After step 1720, the strip detect line is read by the microcontroller, as illustrated in a step 1722. A determination is made as to whether the strip detect line is high or low, as illustrated in a step 1724. If the strip detect is low, then the **low_count** is incremented by one, as illustrated in a step 1726. However, if the strip detect is high, then the **pulse_count** is incremented by one, as illustrated in a step 1728

After step 1728, a determination is made as to whether the pulse_count is equal to four, as illustrated in a step 1730. If the **pulse_count** is less than a predetermined number (e.g., preferably 4), then the process goes back to step 1708. However, if the **pulse_count** is equal to the predetermined number (e.g., preferably 4), then a determination is made as to the number of **high_count** increments and **low_count** increments, as illustrated in a step 1732. If the **high_count** equals four and the **low_count** equals zero, then the strip has been removed, as illustrated in a step 1740. If the **high_count** equals zero and the **low_count** equals four, then the strip is a first type, as illustrated in a step 1738. If the **high_count** equals four and the **low_count** equals four, then the strip is a second type, as illustrated in a step 1734. If the **high_count** and the **low_count** equal any other combination not listed in steps 1734, 1738, and 1740, then the strip type is not determined, as illustrated in a step 1736.

Any implementation of the processes of Figures 15 to 17 can mitigate against the risk that the watchdog timer expires during the worst case scenario where three discrimination attempts are made. This can simply be done by kicking the watchdog timer. Note that the hardware watchdog timer is running during the strip type identification process. Thus, if it were to expire during the strip type identification process then the processor would reset. Note that it is assumed that the strip discrimination line is high upon entry to the detection algorithm and it is returned to the same 'high' state upon exit.

While preferred embodiments of the present invention have been shown and described herein, it will be obvious to those skilled in the art that such embodiments are provided by way of example only. For example, the invention may be applied not only to docking stations and glucose meters, but can also be applied to any electronic device that needs a power supply and that may be re-set such as insulin infusion pump, continuous glucose monitoring system and the like. Moreover, while the various embodiments have been described in relation to blood glucose as an analyte, other analytes can be utilized such as, for example, ketones, cholesterols and the like. Numerous variations, changes, and substitutions will now occur to those skilled in the art without departing from the invention. Various alternatives to the embodiments of the invention described herein may be employed in practicing the invention. It is intended that the following claims define the scope of the invention and that methods and structures within the scope of these claims and their equivalents be covered thereby.

## Claims

1. A method of differentiating between a first type of analyte test strip and second type of analyte test strip with a transistor switch (304) having its gate input connected to a first interrupt of a microcontroller (306) and a drain input of the switch (304) connected to a first contact (212d) and a source input of the switch (304) connected to a ground, the method comprising:
inserting a test strip into a strip port connector (212) so that contact pads of the strip are in electrical connection with the first contact (212d), a second contact (212e), and a third contact (212a) of the strip port connector (212), the third contact being connected to a ground;
detecting via a second interrupt of the microcontroller (306) connected to the second contact (212e) of the strip port connector (212) whether the first contact (212d) is connected to the second contact (212e)formed by a configuration of one or more contact pads of the analyte test strip;
upon detection of the connection of the first (212d) and second (212e) contacts of the strip port connector (212) in common,
turning the switch (304) on and off for a predetermined number of times via the first interrupt;
detecting changes in logic states of the second interrupt; and
identifying the test strip as one at the first type or second type based on a number of high and low logic states of the second interrupt, the second interrupt comprising a connection to a supply voltage via a pull-up resistor.

2. The method of Claim 1 further comprising providing an error message that a first type of test strip has not been mated with the glucose meter where the test strip is of the second type.

3. The method of Claim 1 further comprising instructing a user to input a calibration code where the second type of test strip has been inserted into the glucose meter.

4. The method of Claim 1, in which the first type of test strip includes a continuous conductive track in conductance with the first, second, and third contacts.

5. The method of Claims 2 or 3, in which the second type of test strip has a continuous conductive track in conductance with the first and second contacts, but not the third contact.

6. A glucose meter comprising:
a strip port connector (212) having first (212d), second (212e) and third (212a) contacts, the third contact (212a) connected to a ground;
a transistor switch (304) having a source input, a drain input, and a gate input, the source input connected to a ground, the drain input connected to the first contact (212d) of the strip port connector (212); and
a microcontroller (306) having a first interrupt connected to the gate input of the switch (304) and a second interrupt connected to the second contact (212e) of the strip port connector (212), the microcontroller (306) configured to detect via the second interrupt whether the first contact (212d) is connected to the second contact (212e) upon insertion of a test strip, and configured to open and close the switch (304) with the first interrupt and to measure transitions in a logic state at the second interrupt, in which the second interrupt comprises a connection to a supply voltage via a pull-up resistor.

7. The glucose meter of claim 8, in which the switch comprises one of a MOSFET and FET switch.

8. A glucose measurement system comprising:
a glucose test strip having a plurality of conductive tracks; and
a glucose meter according to claim 6 having a power supply and ground.

## Patentansprüche

1. Verfahren zur Unterscheidung zwischen einem ersten Typ von Analyt-Teststreifen und einem zweiten Typ von Analyt-Teststreifen mit einem Transistorschalter (304), dessen Gate-Eingang mit einem ersten Interrupt eines Mikrocontrollers (306) verbunden ist und wobei ein Drain-Eingang des Schalters (304) mit einem ersten Kontakt (212d) verbunden ist und ein Source-Eingang des Schalters (304) mit einer Masse verbunden ist, wobei das Verfahren aufweist:
Einführen eines Teststreifens in einen Streifenanschlussverbinder (212), so dass die Kontaktstellen des Streifens in elektrischer Verbindung mit dem ersten Kontakt (212d), einem zweiten Kontakt (212e), und einem dritten Kontakt (212a) des Streifenanschlussverbinders (212) sind, wobei der dritte Kontakt mit einer Masse verbunden ist;
Erfassen, über einen zweiten Interrupt des Mikrocontrollers (306), der mit dem zweiten Kontakt (212e) des Streifenanschlussverbinders (212) verbunden ist, ob der erste Kontakt (212d) mit dem zweiten Kontakt (212e) verbunden ist, der durch eine Konfiguration eines oder mehrerer Kontaktstellen des Analyt-Teststreifens gebildet wird;
bei Erfassung der Verbindung des ersten (212d) und zweiten (212e) Kontaktes des Streifenanschlussverbinders (212) gemeinsam,
Ein- und Ausschalten des Schalters (304) für eine vorbestimmte Anzahl von Malen über den ersten Interrupt;
Erfassen von Änderungen von logischen Zuständen des zweiten Interrupts; und
Identifizieren des Teststreifens als einen von dem ersten Typ oder zweiten Typ basierend auf einer Anzahl von hohen und niedrigen logischen Zuständen des zweiten Interrupts, wobei der zweite Interrupt über einen Pullup-Widerstand eine Verbindung zu einer Versorgungsspannung aufweist.

2. Verfahren nach Anspruch 1, ferner aufweisend Bereitstellen einer Fehlermeldung, dass ein erster Typ eines Teststreifens nicht mit dem Blutzuckermessgerät verbunden wurde, wobei der Teststreifen vom zweiten Typ ist.

3. Verfahren nach Anspruch 1, ferner aufweisend Anweisen eines Benutzers, einen Kalibrierungscode einzugeben, wenn der zweite Typ von Teststreifen in das Blutzuckermessgerät eingeführt wurde.

4. Verfahren nach Anspruch 1, bei dem der erste Typ von Teststreifen eine durchgehende Leiterbahn in leitender Verbindung mit dem ersten, zweiten, und dritten Kontakt aufweist.

5. Verfahren nach Anspruch 2 oder 3, bei dem der zweite Typ von Teststreifen eine durchgehende Leiterbahn in leitender Verbindung mit dem ersten und dem zweiten Kontakt, jedoch nicht mit dem dritten Kontakt, aufweist.

6. Blutzuckermessgerät, aufweisend:
einen Streifenanschlussverbinder (212) mit einem ersten (212d), zweiten (212e), und dritten (212a) Kontakt, wobei der dritte Kontakt (212a) mit einer Masse verbunden ist;
einen Transistorschalter (304) mit einem Source-Eingang, einem Drain-Eingang, und einem Gate-Eingang, wobei der Source-Eingang mit einer Masse verbunden ist, wobei der Drain-Eingang mit dem ersten Kontakt (212d) des Streifenanschlussverbinders (212) verbunden ist; und
einen Mikrocontroller (306) mit einem ersten Interrupt, der mit dem Gate-Eingang des Schalters (304) verbunden ist, und einem zweiten Interrupt, der mit dem zweiten Kontakt (212e) des Streifenanschlussverbinders (212) verbunden ist, wobei der Mikrocontroller (306) dazu konfiguriert ist, über den zweiten Interrupt zu erfassen, ob der erste Kontakt (212d) beim Einführen eines Teststreifens mit dem zweiten Kontakt (212e) verbunden ist, und dazu konfiguriert ist, den Schalter (304) mit dem ersten Interrupt zu öffnen und zu schließen, und Übergänge in einem logischen Zustand am zweiten Interrupt, bei dem der zweite Interrupt eine Verbindung zu einer Versorgungsspannung über einen Pullup-Widerstand aufweist, zu messen.

7. Blutzuckermessgerät nach Anspruch 8, bei dem der Schalter einen MOSFET- oder einen FET-Schalter aufweist.

8. Blutzuckermesssystem, aufweisend:
einen Blutzuckermessgerät-Teststreifen mit mehreren Leiterbahnen; und
ein Blutzuckermessgerät nach Anspruch 6 mit einer Stromversorgung und einer Masse.

## Revendications

1. Procédé consistant à établir une distinction entre un premier type de bandelette de test d'analyte et un deuxième type de bandelette de test d'analyte avec un commutateur à transistor (304) ayant son entrée de grille reliée à un premier interrupteur d'un microcontrôleur (306) et une entrée de drain du commutateur (304) reliée à un premier contact (212d) et une entrée de source du commutateur (304) reliée à une terre, le procédé comprenant les étapes suivantes :
insérer une bandelette de test dans un connecteur de port de bandelette (212) de telle sorte que des plots de contact de la bandelette soient en connexion électrique avec le premier contact (212d), un deuxième contact (212e) et un troisième contact (212a) du connecteur de port de bandelette (212), le troisième contact étant relié à une terre ;
détecter, par le biais d'un deuxième interrupteur du microcontrôleur (306) relié au deuxième contact (212e) du connecteur de port de bandelette (212), si le premier contact (212d) est relié au deuxième contact (212e) formé par une configuration d'un ou plusieurs plots de contact de la bandelette de test d'analyte ;
lors de la détection de la connexion des premier (212d) et deuxième (212e) contacts du connecteur de port de bandelette (212) en commun, allumer et éteindre le commutateur (304) un nombre prédéterminé de fois par le biais du premier interrupteur ;
détecter des changements dans des états logiques du deuxième interrupteur ; et
identifier la bandelette de test comme une du premier type ou du deuxième type sur la base d'un nombre d'états logiques hauts et bas du deuxième interrupteur, le deuxième interrupteur comprenant une connexion à une tension d'alimentation par le biais d'une résistance de rappel vers le niveau haut.

2. Procédé de la revendication 1 comprenant en outre l'étape consistant à délivrer un message d'erreur qu'un premier type de bandelette de test n'a pas été couplé avec le glucomètre quand la bandelette de test est du deuxième type.

3. Procédé de la revendication 1 comprenant en outre l'étape consistant à donner l'instruction à un utilisateur de saisir un code d'étalonnage quand le deuxième type de bandelette de test a été inséré dans le glucomètre.

4. Procédé de la revendication 1, dans lequel le premier type de bandelette de test comporte une piste conductrice continue en conduction avec les premier, deuxième et troisième contacts.

5. Procédé des revendications 2 ou 3, dans lequel le deuxième type de bandelette de test a une piste conductrice continue en conduction avec les premier et deuxième contacts, mais pas le troisième contact.

6. Glucomètre comprenant :
un connecteur de port de bandelette (212) ayant des premier (212d), deuxième (212e) et troisième (212a) contacts, le troisième contact (212a) relié à une terre ;
un commutateur à transistor (304) ayant une entrée de source, une entrée de drain et une entrée de grille, l'entrée de source reliée à une terre, l'entrée de drain reliée au premier contact (212d) du connecteur de port de bandelette (212) ; et
un microcontrôleur (306) ayant un premier interrupteur relié à l'entrée de grille du commutateur (304) et un deuxième interrupteur relié au deuxième contact (212e) du connecteur de port de bandelette (212), le microcontrôleur (306) configuré pour détecter par le biais du deuxième interrupteur si le premier contact (212d) est relié au deuxième contact (212e) lors de l'insertion d'une bandelette de test, et configuré pour ouvrir et fermer le commutateur (304) avec le premier interrupteur et pour mesurer des transitions dans un état logique au niveau du deuxième interrupteur, le deuxième interrupteur comprenant une connexion à une tension d'alimentation par le biais d'une résistance de rappel vers le niveau haut.

7. Glucomètre de la revendication 8, dans lequel le commutateur comprend un commutateur MOSFET ou FET.

8. Système de mesure du glucose comprenant :
une bandelette de test de glucose ayant une pluralité de pistes conductrices ; et
un glucomètre selon la revendication 6 ayant une alimentation et une terre.
